# EUROPÄISCHE PATENTSCHRIFT

(11) **EP 1 250 908 B1**
(45) Veröffentlichungstag und Bekanntmachung des Hinweises auf die Patenterteilung: **14.03.2007**
(21) Anmeldenummer: 02008215.2
(22) Anmeldetag: 18.04.2002
(51) Int. Cl.: A61Q 5/10, A61K 8/35, A61K 8/41

(54) **Haarfärbemittel**
Hair dye
Produit pour la coloration des cheveux

(30) Priorität: 18.04.2001 DE 10118889
(43) Veröffentlichungstag der Anmeldung: 23.10.2002
(73) Patentinhaber: KPSS-Kao Professional Salon Services GmbH, 64297 Darmstadt (DE)
(72) Erfinder: Lorenz, Heribert, 64401 Gross-Bieberau (DE)

(56) Entgegenhaltungen:
- EP-A- 0 007 537
- DE-A- 4 434 494
- DATABASE WPI Section Ch, Week 198929 Derwent Publications Ltd., London, GB; Class D21, AN 1989-211458 XP002262720 & JP 01 149708 A (LION CORP) 12. Juni 1989 (1989-06-12)
- DATABASE WPI Section Ch, Week 197030 Derwent Publications Ltd., London, GB; Class D21, AN 1970-52969R XP002262721 & JP 45 021399 B (PIAS KK)

## Beschreibung

Die vorliegende Erfindung betrifft ein Haarfärbemittel auf Basis eines mit Peroxid reagierenden Oxidationsfarbstoff-Systems, das dauerhafte intensive Farbtöne liefert, die in Kombination mit weiteren Entwickler- und Kupplersubstanzen, zur Erzielung weiterer Farbnuancen benutzt werden können.

Die nach wie vor in Haarfärbemitteln meist eingesetzten Entwicklersubstanzen sind 1,4-Diaminobenzol (p-Phenylendiamin) und 1-Methyl-2,5-diaminobenzol (p-Toluylendiamin). Die Verwendung dieser Substanzen wird den farbtechnischen Wünschen der Anwender zwar weitgehend gerecht, es gibt jedoch immer noch Farbnuancen, die dadurch nicht voll erreicht bzw. noch intensiviert werden können.

Es wurde auch bereits vorgeschlagen, diese Lücke durch Verwendung alternativer Entwicklersubstanzen zu schließen. Dies ist in beschränktem Umfang möglich durch den Einsatz von 2-(2,5-Diaminophenyl)ethanol (vgl. EP-A 7537 und EP-B 400 330); jedoch müssen dann Abstriche in der Farbintensität anderer Nuancen hingenommen werden.

Eine weitere befriedigende Lösung dieses Problems wird auch durch den in der EP-A 615 743 beschriebenen Einsatz von 2-(2'-Hydroxyethylamino)-5-aminotoluol bzw. dessen wasserlöslichen Salzen als Entwicklersubstanzen in Haarfärbemitteln erreicht.

DE 44 34 494 A1 offenbart 2-hydroxy-1-ethanon Derivate, beispielweise Acetoin, Hydroxyaceton und Dihydroxyaceton, enthaltende Haarfärbezusammensetzungen. Das Dokument beschreibt die Verwendung von 2-Hydroxy-1-ethanon Derivate in Abwesenheit von Kuppler- und oxidierenden Substanzen.

Selbst dadurch bleiben jedoch noch farbtechnische Wünsche offen.

Die Erfindung geht daher von der Aufgabenstellung aus, ein Haarfärbemittel zu schaffen, das zur Herstellung einer großen Anzahl von Farbtönen geeignet ist und vor allem eine besonders intensive glänzende Färbung bewirkt.

Diese Aufgabe wird dadurch gelöst, daß ein gebrauchsfertiges Haarfärbemittel mindestens ein mit Peroxid reagierendes Oxidationsfarbstoffvorprodukt mindestens eine Kupplersubstanz un ein Oxidationsmittel enthält, wobei das Oxidationsfarbstoffvorprodukt ausgewählt ist aus 1,4-Diaminobenzol und substituierten p-Phenylendiaminen, insbesondere 2,5-Diaminotoluol, 2-n-Propyl- bzw. 2-Ethyl-p-phenylendiamin, 2,6 Dimethyl-p-phenylendiamin, 2-(2,5-Diaminophenyl)ethanol, 1-Amino-4-bis-(2'-hydroxyethyl)-aminobenzol, 2-(2-Hydroxyethylamino)-5-aminotoluol, 4,4'-Diaminodiphenylamin, 4-Aminodiphenylamin, 2-Amino-5-N,N-diethylaminotoluol, 4-Amino-N-ethyl-N-isopropylanilin, 2-Chlor-p-phenylendiamin, 1-β-Hydroxyethyl-2,5-diamino-4-chlorbenzol, 1-β-Hydroxyethyl-2,5-diamino-4-methylbenzol, 2-Methoxy-p-phenylendiamin, N,N-Di-ethyl-p-phenylendiamin, 1-Amino-4-β-methoxyethylaminobenzol, 1-Dimethylamino-4-aminobenzol, 1-Hydroxy-2,5-diamino-4-methylbenzol, 1-Hydroxymethyl-2,5-diaminobenzol, 1,3-Dimethyl-2,5-diaminobenzol, 1,4-Diaminoisopropylbenzol und/oder 1-Amino-4-β-hydroxypropylaminobenzol bzw. deren wasserlöslichen Salzen, in wäßriger Grundlage vorliegt, und 0,01 bis 10 Gew.-% Dihydroxyaceton, berechnet auf die Gesamtzusammensetzung des Mittels, ohne Oxidationsmittel, enthält.

Das erfindungsgemäße Mittel enthält mindestens eine Kupplersubstanz, die ausgewählt sein kann aus Resorcin, 2-Methylresorcin, 4-ChlorResorcin, 2-Amino-4-chlorphenol, 5-Amino-4-methoxy-2-methylphenol, 2-Aminophenol, 3-Aminophenol, 1-Methyl-2-hydroxy-4-aminobenzol, 3-N,N-Dimethylaminophenol, 2,6-Dihydroxy-3,5-dimethoxypyridin, 5-Amino-3-methylphenol, 6-Amino-3-methylphenol, 3-Amino-2-methylamino-6-methoxypyridin, 2-Amino-3-hydroxypyridin, 2-Dimethylamino-5-aminopyridin, 2,6-Diaminopyridin, 1,3-Diaminobenzol, 1-Amino-3-(2'-hydroxyethylamino)benzol, 1-Amino-3-[bis(2'-hydroxyethyl)amino]benzol, α-Naphthol, 4,6-Dichlorresorcin, 1,3-Diamino-toluol, 1-Hydroxynaphthalin, 4-Hydroxy-1,2-methylendioxybenzol, 1,5-Dihydroxynaphthalin, 1,6-Dihydroxynaphthalin, 1,7-Dihydroxynaphthalin, 2,7-Dihydroxynaphthalin, 1-Hydroxy-2-methylnaphthalin, 4-Hydroxy-1,2-methyldioxybenzol, 2,4-Diamino-3-chlorphenol, 5-Amino-2-methoxyphenol und/oder 1-Methoxy-2-amino-4-(2'-hydroxyethylamino)benzol bzw. deren wasserlöslichen Salzen.

Damit soll jedoch der Zusatz weiterer Entwickler- und Kupplersubstanzen keineswegs ausgeschlossen sein.

Bei Anwendung dieser Zusammensetzungen auf Basis einer üblichen Grundlage werden nach der Oxidation mit Peroxid sehr ausdrucksvolle, intensive, dauerhafte Haarfärbungen erhalten, die durch Zusatz entsprechender weiterer Entwickler- und Kupplersubstanzen noch zu anderen Farbnuancen variiert werden können.

Auch die zusätzliche Mitverwendung weiterer, an sich bekannter Entwicklersubstanzen ist möglich. Als solche sind hierbei insbesondere noch Pyrazol und dessen Derivate wie 1-Hydroxyethyl-4,5-diaminopyrazol, 3,4-Diamino-5hydroxypyrazol, 3,5-Diaminopyrazol, 3,5-Diaminopyrazol-1-carboxamid, 3-Amino-5-hydroxypyrazol, 1-Phenyl-2-methylpyrazol, 1-Phenyl-3-methylpyrazol-5-on, 3,5-Dimethylpyrazol, 3,5-Dimethylpyrazol-1-methanol, 3,5-Diamino-1,2,4,-triazol, 2-Aminophenol, 4-Aminophenol und dessen Derivate wie 4-Amino-3-methylphenol, 2-Chlor-4-aminophenol, 2,6-Dichlor-4-aminophenol, 2,4-Diaminophenol, 2,6-Dibrom-4-aminophenol, Hydroxyindole und -indoline sowie Tetraaminopyrimidine, Triaminohydroxypyrimidine, Aminodihydroxypyrimidine, aminosubstituierte Triazine, 5-Aminosalicylsäure und/oder 1,2,4-Triaminobenzol bzw. deren wasserlösliche Salze zu erwähnen.

Die Gesamtkonzentration der Entwicklersubstanzen liegt üblicherweise zwischen etwa 0,05 und 5 %, vorzugsweise 0,1 und 4 %, insbesondere 0,25 bis 3 % Gew.-% der Gesamtzusammensetzung des Haarfärbemittels (ohne Oxidationsmittel), wobei sich die Angaben jeweils auf den Anteil an freier Base beziehen.

Das bevorzugte Gewichtsverhältnis der genannten Entwicklersubstanzen zu den weiteren Entwickler- und Kupplersubstanzen liegt dabei zwischen etwa 1 : 8 bis 8 : 1, vorzugsweise etwa 1 : 5 bis 5 : 1, insbesondere 1 : 2 bis 2 : 1.

Die Kupplersubstanz(en) als Reaktionspartner der Entwicklersubstanz(en) liegen in den erfindungsgemäßen Haarfärbemitteln etwa im gleichen molaren Anteil wie die Entwicklersubstanzen vor, d. h., also in Mengen von 0,01 bis 5,0 %, vorzugsweise 0,05 bis 4 %, insbesondere 0,1 bis 3 Gew.-% der Gesamtzusammensetzung (ohne Oxidationsmittel), wobei sich die Angaben jeweils auf den Anteil an freier Base beziehen.

Der Anteil an Dihydroxyaceton liegt vorzugsweise bei 0,05 bis 5, vor allem 0,25 bis 2,5, insbesondere bei 0,5 bis 2 Gew.-% des Färbemittels (ohne Oxidationsmittelzusammensetzung).

Die erfindungsgemäßen Zusammensetzungen können erwünschtenfalls auch sogenannte Nuanceure zur Feineinstellung des gewünschten Farbtones, insbesondere auch direktziehende Farbstoffe, enthalten.

Solche Nuanceure sind beispielsweise Nitrofarbstoffe wie 2-Amino-4,6-dinitrophenol, 2-Amino-4-nitrophenol, 2-Amino-6-chlor-4-nitrophenol, etc., vorzugsweise in Mengen von etwa 0,05 bis 2,5 %, insbesondere 0,1 bis 1 % Gew.-% der Farbzusammensetzung (ohne Oxidationsmittel).

Die erfindungsgemäßen Haarfärbemittel können die in solchen Mitteln üblichen Grund- und Zusatzstoffe, Konditioniermittel, etc. enthalten, die dem Fachmann aus dem Stand der Technik bekannt und beispielsweise in der Monographie von K. Schrader, "Grundlagen und Rezepturen der Kosmetika", 2. Aufl. (Hüthig Buch Verlag, Heidelberg, 1989), S. 782 bis 815, beschrieben sind. Sie können als Lösungen, Cremes, Gele oder auch in Form von Aerosol-Präparaten vorliegen; geeignete Trägermaterial-Zusammensetzungen sind aus dem Stand der Technik hinreichend bekannt.

Zur Applikation wird das Oxidationsfarbstoff-Vorprodukt mit einem "Oxidationsmittel vermischt. Bevorzugtes Oxidationsmittel ist Wasserstoffperoxid, beispielsweise in 2- bis 12-prozentiger Konzentration.

Es können jedoch auch andere Peroxide wie Harnstoffperoxid und Melaminperoxid eingesetzt werden.

Der pH-Wert des applikationsfertigen Haarfärbemittels, d. h. nach Vermischung mit Peroxid, kann sowohl im schwach sauren, z.B. einem Bereich von 5,5 bis 6,9, im neutralen als auch im alkalischen Bereich, d. h. zwischen pH 7,1 und 11 liegen.

Im folgenden werden verschiedene Ausführungsbeispiele zur Erläuterung der Erfindung gegeben.

| Grundlage | |
|---|---|
| Stearylalkohol | 8,0 (Gew.-%) |
| Kokosfettsäuremonoethanolamid | 4,5 |
| 1,2-Propandiolmono/distearat | 1,3 |
| Kokosfettalkoholpolyglykolether | 4,0 |
| Natriumlaurylsulfat | 1,0 |
| Ölsäure | 2,0 |
| 1,2-Propandiol | 1,5 |
| Na-EDTA | 0,5 |
| Natriumsulfit | 1,0 |
| Eiweißhydrolysat | 0,5 |
| Ascorbinsäure | 0,2 |
| Parfum | 0,4 |
| Ammoniak, 25%ig | 1,0 |
| Ammoniumchlorid | 0,5 |
| Panthenol | 0,8 |
| Wasser | ad 100,00 |

Die erfindungsgemäßen Oxidationsfarbstoff-Kombinationen und Dihydroxyaceton wurden, unter entsprechender Verringerung des Wassergehalts, in diese Grundlage eingearbeitet.

Die Ausfärbungen erfolgten jeweils an Woll-Läppchen und Strähnen aus gebleichtem Menschenhaar, durch Aufbringen einer 1:1-Mischung aus Farbstoff-Vorprodukt und 6%iger Wasserstoffperoxid-Lösung (pH-Wert der Mischung: 9,8) und zwanzigminütiger Einwirkung bei Zimmertemperatur, folgendem Auswaschen und Trocknen.

Es wurden die folgenden Färbungen erzielt:

**1.**

| | 1 | 2 | 3 | 4 | 5 | 6 | 7 | 8 | 9 | 10 | 11 |
|---|---|---|---|---|---|---|---|---|---|---|---|
| p-Phenylendiamin | 0.24% | | | | | | | | | | |
| p-Toluylendiaminsulfiat | | 1,01 | 0,54 | 0,88 | | | | | | | |
| Hydroxyethyl-p-phenylendiaminsulfat | | | | | 0,56 | | | | | | |
| N,N-Bis-Hydroxyethyl-p-phenylendiaminsulfat | | | | | | 0,66 | | | | | |
| 2-(2'-Hydroxyethylamino)-5-aminotoluolsulfat | | | | | | | 0,71 | | | | |
| 2-Arnino-5-diethylamino-toluolhydrochlorid | | | | | | | | 0,48 | | | |
| 4-Amino-N-ethyl-N-iso-propylanilin | | | | | | | | | 0,48 | 0,48 | |
| 1-β-Hydroxyethyl-2,5-di-amino-4-methylbenzol | | | | | | | | | | | 0,60 |
| Resorcin | | | 0,21 | 0,03 | | | | 0,25 | | 0,25 | |
| m-Aminophenol | | | 0,23 | 0,11 | | | 0,13 | | | | |
| m-Phenylendiamin | | | | | | | | | 0,24 | | |
| 4-Amino-2-hydroxytoluol | | 0,55 | 0,04 | | | | | | | | 0,30 |
| 2-Amino-4-hydroxyethyl-aminoanisolsulfat | | | | | | 0,63 | | | | | |
| 2-Amino-3-hydroxypyridin | 0.25% | | | 0,2 | 0,25 | | | | | | |
| 1-Naphthol | | | | 0,16 | | | 0,16 | | | | |

### Färbeergebnisse: Ohne Dihydroxyaceton Mit 0,5% Dihydroxyaceton

| | | |
|---|---|---|
| 1 | Rotbraun | Intensives Rotbraun |
| 2 | Violett | Tiefviolett |
| 3 | Mittelblond | Intensives Mittelblond |
| 4 | Mahagoni | Intensives Mahagoni |
| 5 | Rotbraun | Intensives Rotbraun |
| 6 | Azurblau | Dunkles Azurblau |
| 7 | Marineblau | Dunkles Marineblau |
| 8 | Mattrot | Graubraun |
| 9 | Türkis | Dunkeltürkis |
| 10 | Mattrot | Graubraun |
| 11 | Violett | Tiefviolett |

In die beschriebene Grundlage wurden die folgenden Oxidationsfarbstoffmischungen jeweils mit und ohne 0,5 Gew.-% Dihydroxyaceton eingebracht und der pH-Wert so eingestellt, daß beim Vermischen mit 2%-iger wäßriger H₂O₂-Lösung im Gewichtsverhältnis 1:1 ein pH-Wert der applikationsfertigen Mischung von 6,8 erreicht wurde.

Die Mischungen wurden wiederum jeweils auf Woll-Läppchen und Strähnen aus gebleichtem Menschenhaar aufgebracht, nach 15-minütiger Einwirkung ausgewaschen und getrocknet und die Färbung bewertet.

Es wurde folgendes Ergebnis erzielt:

| Nr. | 1 | 1a | 2 | 2a | 3 | 3a |
|---|---|---|---|---|---|---|
| p-Toluylendiaminsulfat | 1,40 | 1,40 | 0,45 | 0,45 | 1,17 | 1,17 |
| Resorcin | 0,60 | 0,60 | 0,10 | 0,10 | - | - |
| 3-Aminophenol | 0,10 | 0,10 | 0,07 | 0,07 | - | - |
| 2-Amino-4-hydroxyethylaminoanisolsulfat | 0,02 | 0,02 | - | - | - | - |
| 4-Amino-2-hydroxytoluol | - | - | 0,01 | 0,01 | 0,60 | 0,60 |
| 1-Naphthol | - | - | - | - | 0,15 | 0,15 |
| 2,5,6-Triamino-4-hydroxypyrimidinsulfat | - | - | 0,01 | 0,01 | - | - |
| Dihydroxyaceton | 0,50 | - | 0,50 | - | 0,50 | - |
| Färbung | Hellbraun | | Mittelblond | | Violett | |

Die erfindungsgemäßen Zusammensetzungen Nr. 1, 2 und 3 erzielten sämtlich glänzende, intensive Ausfärbungen, die denjenigen der Zusammensetzungen 1a, 2a und 3a deutlich überlegen waren.

Es wurde ein Farbschaumaerosol der folgenden Zusammensetzung hergestellt:

| | | |
|---|---|---|
| p-Toluylendiaminsulfat | | 0,60 (Gew.-%) |
| N,N-Bis(hydroxyethyl)-p-phenylendiaminsulfat | | 0,20 |
| Resorcin | | 0,20 |
| 3-Aminophenol | | 0,15 |
| 1-Naphthol | | 0,05 |
| Natriumsulfit | | 0,10 |
| Ascorbinsäure | | 0,10 |
| Cocamide MEA | | 0,15 |
| Stearamide MEA | | 0,13 |
| Parfum | | 0,15 |
| Hydroxyethylcellulose | | 0,30 |
| Ammoniumchlorid | | 0,25 |
| Cocoamidopropylbetain | | 2,00 |
| Oleth-5 | | 1,00 |
| Cetearylalkohol | | 0,70 |
| Konservierungsmittel | | 0,10 |
| EDTA | | 0,10 |
| Ethanolamin | ad pH | 8,5 |
| Wasser | ad | 100,00 |

100 Gewichtsteile dieser Zusammensetzung wurden mit 6 Gewichtsteilen eines handelsüblichen Treibmittelgemisches aus Propan/Butan in eine mit einem Schaumventil versehenen Aerosoldose abgefüllt.

Einem Teil der Zusammensetzung wurden 0,7 Gew.-% Dihydroxyaceton zugesetzt.

In einem Halbseitenversuch wurde auf die nassen Haare einer Kopfhälfte die Zusammensetzung ohne, auf die andere Kopfhälfte die Zusammensetzung mit Dihydroxyaceton gleichmäßig aufgebracht.

Nach 15-minütiger Einwirkung wurde gespült, getrocknet und beide Haarhälften miteinander verglichen.

Die mit der erfindungsgemäßen, Dihydroxyaceton enthaltenden Zusammensetzung behandelte Haarhälfte wies eine glänzende mittelblonde Färbung auf, während die mit der Zusammensetzung nach dem Stand der Technik behandelte Haarhälfte eine deutlich blässere Hellblondfärbung zeigte.

## Patentansprüche

1. Gebrauchsfertige zum Färben von menschlichen Haaren, enthaltend mindestens ein Oxidationsfarbstoffvorprodukt, ausgewählt aus 1,4-Diaminobenzol und substituierten p-Phenylendiaminen bzw. deren wasserlöslichen Salzen, mindestens eine Kupplersubstanz und ein Oxidationsmittel in wäßriger Grundlage, **gekennzeichnet durch** einen Gehalt an 0,01 bis 10 Gew.-% Dihydroxyaceton, berechnet auf die Gesamtzusammensetzung des Mittels ohne Oxidationsmittel.

2. Mittel nach Anspruch 1, enthaltend als substituierte p-Phenylendiamine 2,5-Diaminotoluol, 2-n-Propyl- bzw. 2-Ethyl-p-phenylendiamin, 2,6-Dimethyl-p-phenylendiamin, 2-(2,5-Diaminophenyl)ethanol, 1-Amino-4-bis-(2'-hydroxyethyl)-aminobenzol, 2-(2-Hydroxyethylamino)-5-aminotoluol, 4,4'-Diaminodiphenylamin, 4-Aminodiphenylamin, 2-Amino-5-N,N-diethylaminotoluol, 4-Amino-N-ethyl-N-isopropylanilin, 2-Chlor-p-phenylendiamin, 1-β-Hydroxyethyl-2,5-diamino-4-chlorbenzol, 1-β-Hydroxyethyl-2,5-diamino-4-methylbenzol, 2-Methoxy-p-phenylendiamin, N,N-Diethyl-p-phenylendiamin, 1-Amino-4-β-methoxyethyl-aminobenzol, 1-Dimethylamino-4-aminobenzol, 1-Hydroxy-2,5-diamino-4-methylbenzol, 1-Hydroxymethyl-2,5-diaminobenzol, 1,3-Dimethyl-2,5-diaminobenzol, 1,4-Diaminoisopropylbenzol und/oder 1-Amino-4-β-hydroxypropylaminobenzol bzw. deren wasserlöslichen Salzen.

3. Mittel nach Ansprüchen 1 und 2, enthaltend mindestens eine Kupplersubstanz, ausgewählt aus Resorcin, 2-Methylresorcin, 4-Chlorresorcin, 2-Amino-4-chlorphenol, 5-Amino-4-methoxy-2-methylphenol, 2-Aminophenol, 3-Aminophenol, 1-Methyl-2-hydroxy-4-aminobenzol, 3-N,N-Dimethylaminophenol, 2,6-Dihydroxy-3,5-dimethoxypyridin, 5-Amino-3-methylphenol, 6-Amino-3-methylphenol, 3-Amino-2-methylamino-6-methoxypyridin, 2-Amino-3-hydroxypyridin, 2-Dimethylamino-5-aminopyridin, 2,6-Diaminopyridin, 1,3-Diaminobenzol, 1-Amino-3-(2'-hydroxy-ethylamino)benzol, 1-Amino-3-[bis(2'hydroxyethyl)amino]benzol, α-Naphthol, 4,6-Dichlorresorcin, 1,3-Diaminotoluol, 1-Hydroxynaphthalin, 4-Hydroxy-1,2-methylendioxybenzol, 1,5-Dihydroxynaphthalin, 1,6-Dihydroxynaphthalin,1,7-Dihydroxynaphthalin, 2,7-Dihydroxynaphthalin, 1-Hydroxy-2-methylnaphthalin, 4-Hydroxy-1,2-methyleridioxybenzol, 2,4-Diamino-3-chlorphenol, 5-Amino-2-methoxyphenol und/oder 1-Methoxy-2-amino-4-(2'-hydroxyethylamino)benzol bzw. deren wasserlöslichen Salzen.

4. Mittel nach einem oder mehreren der Ansprüche 1 bis 3, enthaltend 0,05 bis 5 Gew.-% Dihydroxyaceton, bezogen auf die Gesamtzusammensetzung ohne Oxidationsmittel

5. Verwendung von Dihydroxyaceton in Haarfärbemitteln auf Basis mindestens eines Oxidationsfarbstoffvorprodukts, ausgewählt aus 1,4-Diaminobenzol und substituierten p-Phenylendiaminen bzw. deren wasserlöslichen Salzen, mindestens eine Kupplersubstanz und ein Oxidationsmittel.

## Claims

1. Ready-to-use composition for the dyeing of human hair, comprising at least one oxidation dyestuff precursor, selected from 1,4-diaminobenzene and substituted p-phenylenediamines or the water-soluble salts thereof, at least one coupling substance and one oxidizing agent in aqueous carrier, **characterized by** a content of 0.01 % to 10 % by weight of dihydroxy acetone, calculated to the total composition without oxidizing agent.

2. Composition according to claim 1, comprising as substituted p-phenylenediamines 2,5-diamino-toluene, 2-n-propyl or 2-ethyl-p-phenylene-diamine, 2,6-dimethyl-p-phenylene- diamine, 2-(2,5-diaminophenyl) ethanol, 1-amino -4-bis-(2'-hydroxyethyl)amino- benzene, 2-(2-hydroxyethyl amino)-5-aminotoluene, 4,4'-diaminodiphenylamine, 4-aminodiphenylamine, 2-amino-5-N,N-diethyl amino-toluene, 4-amino-N-ethyl-N-isopropyl aniline, 2-chloro-p-phenylenediamine, 1-β-hydroxyethyl-2,5-diamino-4-chlorobenzene, 1-β-hydroxyethyl-2,5-diamino-4-methyl benzene, 2-methoxy-p-phenylenediamine, N,N-diethyl-p-phenylenediamine, 1-amino-4-β-methoxyethyl aminobenzene, 1-dimethyl-amino-4-aminobenzene, 1-hydroxy-2,5-diamino-4-methyl benzene, 1-hydroxymethyl-2,5-diaminobenzene, 1,3-dimethyl-2,5-diaminobenzene, 1,4-diamino isopropyl benzene and/or 1-amino-4-β-hydroxypropyl aminobenzene or the water-soluble salts thereof.

3. Composition according to claims 1 and 2, comprising at least one coupling substance selected from resorcinol, 2-methyl resorcinol, 4-chlororesorcinol, 2-amino-4-chlorophenol, 5-amino-4-methoxy-2-methylphenol, 2-aminophenol, 3-aminophenol, 1-methyl-2-hydroxy-4-aminobenzene, 3-N,N-dimethyl aminophenol, 2,6-dihydroxy-3,5-dimethoxypyridine, 5-amino-3-methylphenol, 6-amino-3-methylphenol, 3-amino-2-methylamino-6-methoxypyridine, 2-amino-3-hydroxypyridine, 2-dimethyl-amino-5-aminopyridine, 2,6-diaminopyridine, 1,3-diaminobenzene, 1-amino-3-(2'-hy-droxyethylamino)benzene, 1-amino-3-[bis(2'-hydroxyethyl) amino]benzene, α-naphthol, 4,6-dichlororesorcinol, 1,3-diamino-toluene, 1-hydroxy naphthalene, 4-hydroxy-1,2-methylenedioxy benzene, 1,5-dihydroxy naphthalene, 1,6-dihydroxy naphthalene, 1,7-dihydroxy naphthalene, 2,7-dihydroxy naphthalene, 1-hydroxy-2-methyl naphthalene, 4-hydroxy-1.2-methyldioxy benzene, 2,4-diamino-3-chlorophenol, 5-amino-2-methoxyphenol and/or 1-methoxy-2-amino-4-(2'-hydroxyethyl amino)benzene or the water-soluble salts thereof

4. Composition according to one or more of claims 1 to 3, comprising 0.05 % to 5 % by weight of dihydroxy acetone, calculated to the total composition without oxidizing agent.

5. Use of dihydroxy acetone in hair coloration compositions on the basis of at least one oxidation dyestuff precursor selected from 1,4-diaminobenzene and substituted p-phenylenediamines or the water-soluble salts thereof, at least one coupling substance and one oxidizing agent.

## Revendications

1. Produit prêt à l'emploi pour la coloration des cheveux, contenant au moins un précurseur de colorant d'oxydation, choisi parmi le 1,4-diaminobenzène et des p-phénylènediamines substituées, ou leurs sels solubles dans l'eau, au moins une substance de couplage et un agent oxydant, dans une base aqueuse, **caractérisé par** une teneur de 0,01 à 10 % en poids de dihydroxyacétone, calculés sur, la composition totale du produit sans agent oxydant.

2. Produit selon la revendication 1, contenant comme p-phénylènediamines substituées du 2,5-diaminotoluène, de la 2-n-propyl-, ou de la 2-éthyl-p-phénylènediamine, de la 2,6-diméthyl-p-phénylènediamine, du 2-(2,5-diaminophényl)éthanol, du 1-amino-4-bis-(2'-hydroxyéthyl)-aminobenzène, du 2-(2-hydroxyéthylamino)-5-aminotoluène, de la 4,4'-diaminodiphénylamine, de la 4-aminodiphénylamine, du 2-amino-5-N,N-diéthylaminotoluène, de la 4-amino-N-éthyl-N-isopropylaniline, de la 2-chloro-p-phénylènediamine, du 1-β-hydroxyéthyl-2,5-diamino-4-chlorobenzène, du 1-β-hydroxyéthyl-2,5-diamino-4-méthylbenzène, de la 2-méthoxy-p-phénylènediamine, de la N,N-diéthyl-p-phénylènediamine, du 1-amino-4-β-méthoxyéthyl-aminobenzène, du 1-diméthylamino-4-aminobenzène, du 1-hydroxy-2,5-diamino-4-méthylbenzène, du 1-hydroxyméthyl-2,5-diaminobenzène, du 1,3-diméthyl-2,5-diaminobenzène, du 1,4-diamino-isopropylbenzène, et/ou du 1-amino-4-β-hydroxypropylaminobenzène, ou leurs sels solubles dans l'eau.

3. Produit selon les revendications 1 et 2, contenant au moins une substance de couplage, choisie parmi la résorcine, la 2-méthylrésorcine, la 4-chlororésorcine, le 2-amino-4-chlorophénol, le 5-amino-4-méthoxy-2-méthylphénol, le 2-aminophénol, le 3-aminophénol, le 1-méthyl-2-hydroxy-4-aminobenzol, le 3-N,N-diméthylaminophénol, la 2,6-hydroxy-3,5-diméthoxypyridine, le 5-amino-3-méthylphénol, le 6-amino-3-méthylphénol, la 3-amino-2-méthylamino-6-méthoxypyridine, la 2-amino-3-hydroxypyridine, la 2-diméthylamino-5-aminopyridine, la 2,6-diaminopyridine, le 1,3-diaminobenzène, le 1-amino-3-(2'-hydroxyéthylamino)benzène, le 1-amino-3-[bis(2'-hydroxyéthyl)amino]benzène, l'α-naphtène, la 4,6-dichlororésorcine, le 1,3-diaminotoluène, le 1-hydroxynaphtalène, le 4-hydroxy-1,2-méthylènedioxybenzène, le 1,5-dihydroxynaphtalène, le 1,6-dihydroxynaphtalène, le 1,7-dihydroxynaphtalène, le 2,7-dihydroxynaphtalène, le 1-hydroxy-2-méthylnaphtalène, le 4-hydroxy-1,2-méthylènedioxybenzène, le 2,4-diamino-3-chlorophénol, le 5-amino-2-méthoxyphénol et/ou le 1-méthoxy-2-amino-4-(2'-hydroxyéthylamino)benzène, ou leurs sels solubles dans l'eau.

4. Produit selon l'une ou plusieurs des revendications 1 à 3, contenant 0,05 à 5 % en poids de dihydroxyacétone, rapportés à la composition totale sans agent oxydant.

5. Utilisation de la dihydroxyacétone dans des produits pour la coloration des cheveux, à base d'au moins un précurseur de colorant d'oxydation, choisi parmi le 1,4-diaminobenzène et des p-phénylènediamines substituées, ou leurs sels solubles dans l'eau, au moins une substance de couplage et un agent oxydant.
